# EUROPEAN PATENT SPECIFICATION

(11) **EP 2 910 264 B1**
(45) Date of publication and mention of the grant of the patent: **30.05.2018**
(21) Application number: 14305238.9
(22) Date of filing: 20.02.2014
(51) Int. Cl.: A61M 5/145

(54) **Holding device for a syringe pump**
Haltevorrichtung für eine Spritzenpumpe
Dispositif de maintien pour une pompe à seringue

(43) Date of publication of application: 26.08.2015
(73) Proprietor: Fresenius Vial SAS, 38590 Brézins (FR)
(72) Inventor: Traversaz, Philippe, 38140 Saint-Blaise Du Buis (FR); Barthelemy, Roman, 38320 Eybens (FR); Perrigouard, Florie, 38700 La Tronche (FR); Thomas, Fabien, 38300 Meyrie (FR)
(74) Representative: Kusche, Robert

(56) References cited:
- EP-A1- 0 567 962
- WO-A1-2014/100658
- FR-A1- 2 889 961
- JP-A- 2004 305 361
- US-A- 5 176 646

## Description

The invention relates to holding device for a syringe pump according to the preamble of claim 1.
A holding device with the features of the preamble of claim 1 is known from EP 0 567 962 A1. A further prior art holding device in the sense of Art. 54(3) EPC is known from WO 2014/100658 A1. A variety of mechanisms is known for fixing a syringe to a syringe pump. For example, a syringe pump may have a receiving section for receiving the barrel of a syringe, wherein a latchable clamp member is used to fix the syringe barrel to the receiving section as disclosed in US 3 964 139 B2.

Further examples of holding devices are disclosed in US 5 176 646 A or FR 2889961 A1.

It is an object of the invention to provide a holding device for a syringe pump that securely fixes a syringe barrel to a syringe pump and at the same time keeps the risk that the syringe will be damaged by the holding device as low as possible.

According to the invention, a holding device for a syringe pump is provided, the holding device being configured for holding the barrel of a syringe and comprising:
- at least one holding member which is rotatable from an open into a closed position; and
- a blocking mechanism interacting with the holding member in such a way that it permits a rotation of the holding member from the open into the closed position and blocks a rotation from the closed into the open position, wherein
- a clutching mechanism allowing the holding member to be disengaged from the blocking mechanism such that the holding member can be rotated from the closed into the open position, and
- the blocking mechanism comprises a one-way bearing via which the holding member is rotatably mounted to the receiving portion of the syringe pump, wherein the holding member is connected to a shaft that is rotatably mounted to a receiving portion of the syringe pump for receiving the syringe barrel, wherein an axis of the shaft and an axis of the receiving portion are parallel to each other

The blocking and the clutching mechanism of the holding device according to the invention permit to hold the syringe barrel with the necessary holding force without having to use a spring of high spring constant for pre-tensioning the holding member against the syringe barrel. Rather, a spring with a rather small spring constant can be used such that the risk of damaging the syringe barrel (particularly of smaller syringes) when moving the holding member from the open into the closed position is reduced.

Nevertheless, the syringe barrel can be securely fixed to the syringe pump. In particular, the blocking mechanism may be configured in such a way that the EN 60601-2-24 Ed. 3 standard is met. For example, the blocking mechanism secures the syringe barrel so that a force (in any direction) of at least 15 N exerted to the syringe tip (by the extension line) for at least 15 s does not detach the syringe from the syringe pump.

For example, the holding device may be configured in such a way that the syringe barrel is secured between the holding member and a section of a receiving portion of the syringe pump for receiving the syringe barrel. However, it is also conceivable that the holding device comprises two rotatable holding members, wherein the syringe barrel is secured between the two holding members and wherein both the blocking and the clutching mechanism interact with both holding members.

The one-way bearing allows the holding member to be pivoted into the closed position, but blocks a rotation in the opposite direction, i.e. a rotation of the holding member towards the open position. For example, the holding member is connected to a shaft about which it can be rotated, wherein the shaft is rotatably mounted to the receiving portion of the syringe pump via the one-way bearing.

Further, the clutching mechanism may comprise a first part fixedly connected to the receiving portion of the syringe pump and a second part interacting with the first part, the second part being coupled (e.g. connected) to the holding member in such way that it can be disengaged from the first part by moving away the holding member from the first part. In particular, the holding member will be disengaged from the blocking mechanism by disengaging the second part from the first part. Thus, the blocking mechanism can be bypassed by disengaging the two parts of the clutching mechanism, thereby allowing the holding member to be rotated from the closed into the open position.

For example, the second part of the clutching mechanism is coupled to the holding member in such a way that it will follow a linear movement of the holding member. Thus, the second part of the clutching mechanism can be disengaged from the first part by moving the holding member away from the first part along an axis of a shaft via which the holding member is mounted to a receiving portion of the syringe pump for receiving the syringe barrel. That is, in the closed position the holding member can be uncoupled from the blocking mechanism by linearly moving the holding member away from the first part of the clutching mechanism.

Further, the blocking mechanism may comprise a one-way bearing (as already set forth above), the one-way bearing may be fixedly connected to the second part of the clutching mechanism. Thus, if the two parts of the clutching mechanism are in engagement with one another, the one-way bearing will be fixedly connected to the receiving portion of the syringe pump (via the first and the second part of the clutching mechanism) and will only allow the rotation of the holding member in one direction (towards the closed position). However, if the two parts of the clutching mechanism are disengaged, the one-way bearing is not fixed to the receiving portion of the syringe pump anymore and therefore will be rotated together with the holding member when the holding member is rotated from the closed position into the open position and thus cannot block this rotation anymore.

The holding member may comprise a first and a second lever element, wherein the first lever element can be rotated relative to the second lever element and the holding device is to be arranged on the receiving portion of the syringe pump in such a way that when the first lever element is rotated, a force is exerted on the holding member that generates a linear movement of the holding member. This linear movement of the holding member will disengage the second part from the first part of the clutching mechanism and thus will disengage the holding member from the blocking mechanism.

The first and the second part of the clutching mechanism may realize a form-fitted clutch, e.g. a jaw clutch, wherein, for example, the first and the second part each comprise a toothing that can be brought into engagement with one another. Further, the first and the second part of the clutching mechanism may form a one-way clutch. In that embodiment the clutching mechanism may thus also form the blocking mechanism because already the clutching mechanism may block the rotation of the holding member into the open position.

According to another embodiment of the invention, the holding device comprises a spring that has the tendency to rotate the holding member into the closed position; e.g. the spring is configured to bias the holding member against the syringe barrel. The spring may be a coil spring surrounding the above-mentioned shaft connected to the holding member.

The invention also relates to a syringe pump comprising a holding device as described above.

Embodiments of the invention will be described in more detail hereinafter with reference to the drawings, in which
- Fig. 1: shows an exploded view of a holding device for a syringe pump according to an embodiment of the invention;
- Fig. 2: shows a perspective view of a detail of the holding device of Fig. 1;
- Fig. 3: depicts a sectional view of the holding device of Fig. 1;
- Fig. 4A: shows a spring section of a holding device according to the invention;
- Fig. 4B: shows another view of the spring section depicted in Fig. 4A; and
- Fig. 5: illustrates a syringe pump with a holding device according to the invention.

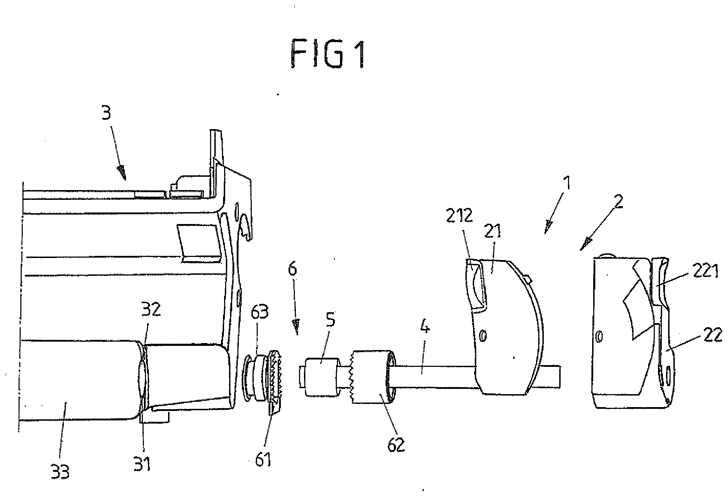

The holding device 1 for a syringe pump according to the invention shown in Figures 1 to 3 comprises a holding member in the form of a two-part rotatable lever 2. The rotatable lever 2 is configured to hold the barrel of a syringe (not shown) on to a receiving portion 3 of the syringe pump, wherein the lever 2 is connected to a shaft 4 about which it can be rotated from an open position to a closed position.

In the open position the lever 2 is positioned in a distance from the receiving portion 3 of the syringe pump such that the syringe barrel can be arranged on the receiving portion 3. After having positioned the syringe barrel on the receiving portion 3 the lever 2 is moved towards the syringe barrel into the closed position in which the syringe barrel is secured between the lever 2 and the receiving portion 3. In particular, the lever 2 bears against the syringe barrel in the closed position.

The holding device 1 further comprises a blocking mechanism that interacts with the rotatable lever 2 in such a way that it permits a rotation of lever 2 from the open into the closed position but blocks a rotation from the closed into the open position. The blocking mechanism according to the embodiment shown in Fig. 1 is realized by a one-way bearing 5 via which the shaft 4 and thus the rotatable lever 2 is rotatably mounted at the receiving portion 3. The one-way bearing 5 permits a rotation only in the clockwise direction (when looking at the bearing from the lever side) such that once the lever 2 is rotated into the closed position it will remain in that position.

However, in particular for unloading the syringe from the syringe pump, the holding device 1 comprises a clutching mechanism 6 that allows the lever 2 to be disengaged from the blocking mechanism. For this, the clutching mechanism 6 comprises a first part in the form of a fixed toothed part 61 and a second part in the form of a mobile toothed part 62. The fixed toothed part 61 is fixedly connected to the receiving portion 3 of the syringe pump, wherein the fixed toothed part 61 may be (e.g. integrally) connected to a spacer 63 inserted into an opening 31 of the receiving portion 3. It is noted that in the assembled state of the holding device also the shaft 4 will be inserted through the opening 31 into a housing section 33 of the receiving portion 3.

The mobile toothed part 62 is fixedly connected to the one-way bearing 5, wherein the mobile toothed part 62 is a hollow-cylindrical part arranged on the shaft 4 in such a way that it surrounds the one-way bearing 5. Thus, if the toothing of the mobile toothed part 62 engages the toothing of the fixed toothed part 61, the one-way bearing 5 will be fixedly coupled to the receiving portion 3 and thus will operate as described above. However, if the toothing of the mobile toothed part 62 is disengaged from the toothing of the fixed toothed part 61, the one-way bearing 5 is not coupled to the receiving portion 3 anymore and thus will be able to rotate along with the shaft 4 if the shaft 4 is rotated in the counterclockwise direction, i.e. if the lever 2 is pivoted from the closed into the opened position. Thus, if the mobile toothed part 62 is moved away from the fixed toothed part 61 along the axis of shaft 4, the one-way bearing 5 cannot block the rotation of the lever 2 into the opened position anymore.

For generating a linear movement of the mobile toothed part 62 away from the fixed toothed part 61, the lever 2 comprises a movable lever part 21 shifting away the mobile toothed part 62 from the receiving portion 3 and more particularly from the front face 32 of the receiving portion 3. It is noted that the mobile toothed part 62 part will follow a translational movement of the lever 2. That is, the mobile toothed part 62 will rotate along with the lever 2 when the lever 2 is rotated from the closed position into the open position, i.e. when the linear movement was carried out beforehand.

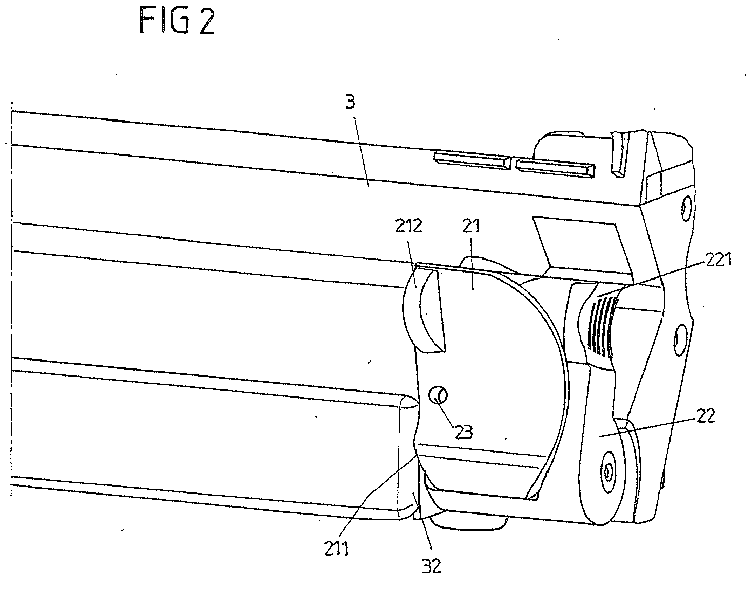

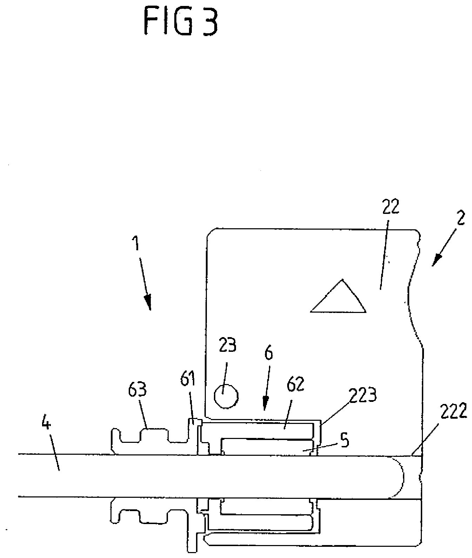

The movable lever part 21 is connected to a base lever part 22 that will bear against the syringe barrel when the lever 2 is in the closed position. More particularly, the movable lever part 21 is connected to the base lever part 22 via a pin 23 (see Figure 2) such that the movable lever part 21 can be pivoted relative to the base lever part 22 around the pin 23. Further, in the assembled state (see Fig. 2 and 3) of the syringe pump the lever 2 is arranged adjacent a front face 32 (comprising the opening 31) of a housing section 33 of the receiving portion 3 of the syringe pump such that if the movable lever part 21 is rotated in clockwise direction (with respect to Fig. 2) a lower bearing portion 211 of the movable lever part 21 will get in contact with the front face 32 of the receiving portion 3. Thus, a counter force will be exerted on the movable lever part 21 shifting it away from the front face 32 of the receiving portion 3 together with the base lever part 22. Thus, as the mobile toothed part 62 will follow this longitudinal movement of the lever 2, it will be disengaged from the fixed toothed part 61 such that the lever 2 is unblocked and can be rotated from the closed into the open position for loading or unloading a syringe into/from the syringe pump.

For generating the described rotation of the movable lever part 21 it comprises a handle 212, wherein the base lever part 22 comprises a similar handle 221 such that by pressing the handles 212, 221 together as indicated by the arrows in Figure 2 (e.g. using the thumb and another finger) the movable lever part 21 will be rotated and the lever 2 as a whole (and in fact the whole holding device 1 except the fixed toothed part 61 and the spacer 63) will be displaced away from the front face 32 of the receiving portion 3 (i.e. to the right in Fig. 2) to disengage the two parts 61, 62 of the clutching mechanism 6 from one another.

As further illustrated in Fig. 3, the base lever part 22 comprises a recess 222 (e.g. a bore hole) through which the shaft 4 extends (the movable lever part 21 being omitted in Fig. 3). A further recess 223 is provided for receiving the one-way bearing 5 and the mobile toothed part 62. The lever part 22 in particular is rigidly mounted to the shaft 4 (e.g. the lever part 22 is generated by overmolding the shaft 4).

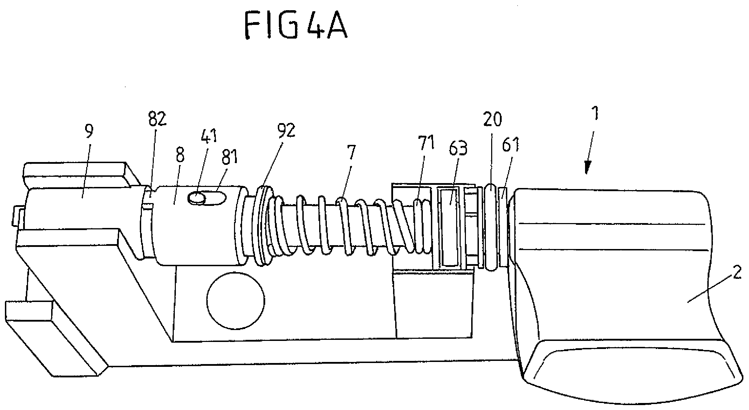

As illustrated in Fig. 4A, the holding device 1 according to the invention may comprise a spring 7 that has the tendency to rotate the lever 2 in the closed position and to bias the lever 2 against the syringe barrel. However, since the lever 2 is secured in the closed position by the blocking mechanism of the holding device 1 and not by means of the spring 7, a spring with a rather low spring constant could be used to avoid damage to the syringe barrel.

The spring 7 is a coil spring surrounding the shaft 4, wherein an end 71 of the spring 7 is connected to the spacer 63 (that with a side facing away from the spring 7 forms the fixed toothed part 61 of the clutching mechanism 6 as set forth above). The shaft 4 and the spring 7 in the assembled state of the syringe pump will be arranged in the housing section 33 (see Fig. 1) of the receiving portion 3 of the syringe pump, wherein a ring seal 20 is arranged on the spacer 63 that seals the section of the holding device 1 that is located within the housing section 33.

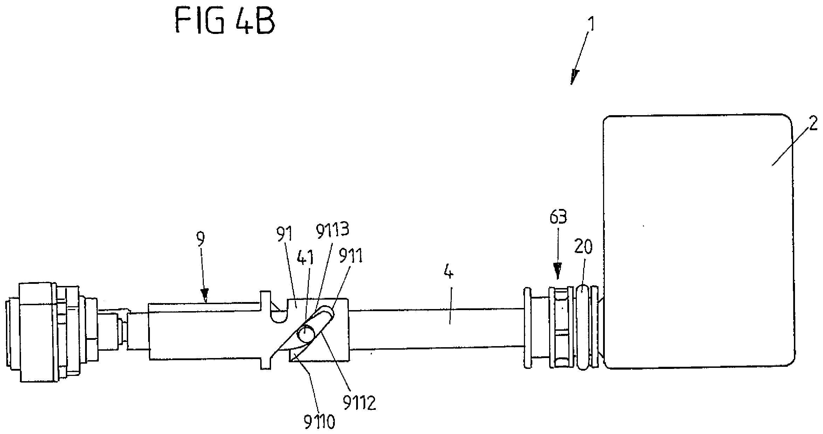

Further, the shaft 4 comprises a radially extending pin 41 engaging a longitudinal opening 81 of a captivity tube 8. The holding device 1 further comprises a cam tube 9 partially covered by the captivity tube 8 in Fig. 4A. Fig. 4B also illustrates the spring section shown in Fig. 4A, wherein some components (such as the captivity tube 8 and the spring 7) have been omitted for the sake of clarity.

According to Fig. 4B, a section 91 of the cam tube 9, which is covered by the captivity tube 8, comprises a slit-like opening 911 receiving the pin 41 of shaft 4. The opening 911 extends in a plane that is inclined (towards lever 2) relative to the axis of shaft 4. When the shaft 4 (i.e. the lever 2) is rotated, the pin 41 will push against an edge 9110 of the opening 911 such that a translation of the cam tube 9 parallel to the axis of shaft 4 is generated. In particular, the cam tube 9 will be moved towards the lever 2 when the lever 2 is rotated into the open position (shown in Fig. 4A), thereby compressing the spring 7. Conversely, the cam tube 9 moves away from the lever 2 when the lever 2 is rotated into the closed position. The captivity tube 8 is used to prevent the pin 41 from disengaging from opening 911 and may be coupled to the cam tube 9 (to the left part of the cam tube 9) via a protrusion 82 engaging a notch of the cam tube 9.

More particularly, the pin 41 will engage a lower portion 9112 of the edge 9110 when the lever 2 is rotated into the open position (as shown in Fig. 4A), thereby pushing the cam tube 9 towards the lever 2 against the spring 7 (not shown in Fig. 4B). Conversely, when the lever 2 is rotated into the closed position (i.e. upwards in Fig. 4A until the position shown in Fig. 4B is reached) using the support of spring 7, the pin 41 will engage an upper portion 9113 of edge 9110, thereby pushing the cam tube 9 away from the lever 2.

A washer 92 may be arranged between the captivity tube 8 and spring 7.

It is noted that according to the embodiment shown in Fig. 4A, 4B, the lever 2 is a one-part piece, wherein the lever 2 could be decoupled from the blocking mechanism (not shown in Fig. 4A, 4B) by moving the lever 2 away from the spacer 63 such that the mobile toothed part 62 will be disengaged from the fixed toothed part 61 as explained above. In that case, the spring 7 will exert a restoring force on the lever 2 such that the lever 2 will have the tendency to move back again towards the spacer 63 (i.e. towards the housing section 33 of the receiving portion 3 of the syringe pump) such that the mobile toothed part 62 has the tendency to engage the fixed toothed part 61 and thus to activate the blocking mechanism again. It is of course also possible that the lever 2 is a two-piece part as shown in Fig. 1 to 3, i.e. the spring mechanism illustrated in Fig. 4A can be used with the embodiment of Fig. 1 to 3.

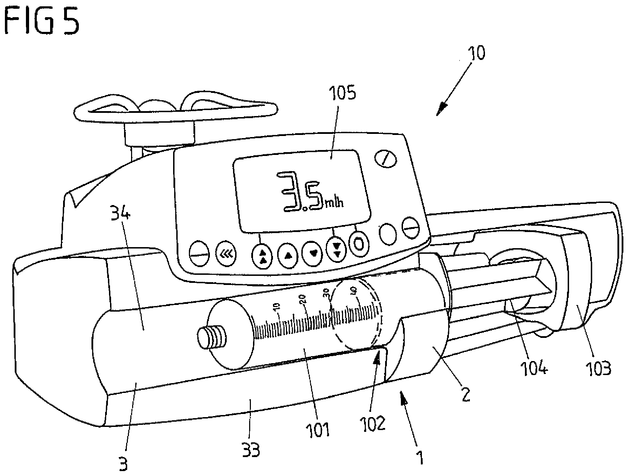

Figure 5 illustrates a syringe pump 10 comprising a holding device 1 according to the invention (e.g. the holding device shown in Fig. 1 to 3). The barrel 101 of a syringe 102 is arranged in the receiving section 3 of the syringe pump 10, wherein the lever 2 of the holding device 1 is about to move into the closed position for securing the syringe barrel 101 between the lever 2 and a curved sidewall 34 of the receiving section 3. The other components of the holding device are arranged within the housing section 33 of the receiving section 3 or within an opening of the lever 2 (see, for example, Fig. 2).

The syringe pump 10 further comprises a display 105 and a movable gripper 103 for moving the piston 104 of the syringe 102 as in principle known in the art.

### Reference signs

- 1: holding device
- 2: rotatable lever
- 3: receiving section
- 4: shaft
- 5: one-way bearing
- 6: clutching mechanism
- 7: spring
- 8: captivity tube
- 9: cam tube
- 10: syringe pump
- 20: ring seal
- 21: movable lever part
- 22: base lever part
- 23: pin
- 31: opening
- 32: front face
- 33: housing section
- 41: radial pin
- 61: fixed toothed part
- 62: mobile toothed part
- 63: spacer
- 71: spring end
- 81: opening
- 82: protrusion
- 91: section cam tube
- 92: washer
- 101: syringe barrel
- 102: syringe
- 103: gripper
- 104: piston
- 105: display
- 211: lower bearing portion
- 212, 221: handle
- 222: recess
- 223: further recess
- 911: opening
- 9110: edge
- 9112: lower portion of edge
- 9113: upper portion of edge

## Claims

1. Holding device for a syringe pump, the holding device (1) being configured for holding the barrel (101) of a syringe (102) and comprising:
- at least one holding member (2) which is rotatable from an open into a closed position; and
- a blocking mechanism interacting with the holding member (2) in such a way that it permits a rotation of the holding member (2) from the open into the closed position and blocks a rotation from the closed into the open position,
**characterized by**
a clutching mechanism (6) allowing the holding member (2) to be disengaged from the blocking mechanism such that the holding member (2) can be rotated from the closed into the open position, and that the blocking mechanism comprises a one-way bearing (5) via which the holding member (2) is rotatably mounted to a receiving portion (3) of the syringe pump (10) for receiving the syringe barrel (101),
wherein
the holding member (2) is connected to a shaft (4) that is rotatably mounted to a receiving portion (3) of the syringe pump (10) for receiving the syringe barrel (101),
wherein an axis of the shaft (4) and an axis of the receiving portion (3) are parallel to each other.

2. Holding device as claimed in claims 1, wherein the shaft (4) is rotatably mounted to the receiving portion (3) of the syringe pump (10) via the one-way bearing (5).

3. Holding device as claimed in one of the preceding claims, wherein the clutching mechanism (6) comprises a first part (61) fixedly connected to a receiving portion (3) of the syringe pump (10) for receiving the syringe barrel (101) and a second part (62) interacting with the first part (61), the second part (62) being coupled to the holding member (2) in such way that it can be disengaged from the first part (61) by moving the holding member (2) away from the first part (61).

4. Holding device as claimed in claim 3, wherein the holding member (2) can be disengaged from the blocking mechanism by disengaging the second part (62) from the first part (61).

5. Holding device as claimed in claims 1 and 4, wherein the second part (62) can be disengaged from the first part (61) by moving the holding member (2) away from the first part (61) along the axis of the shaft (4).

6. Holding device as claimed in one of the claims 3 to 5 as far as dependent on claim 1, wherein the one-way bearing (5) is fixedly connected to the second part (62) of the clutching mechanism (6).

7. Holding device as claimed in one of the claims 3 to 6, wherein the second part (62) of the clutching mechanism (6) is coupled to the holding member (2) in such a way that it will follow a linear movement of the holding member (2).

8. Holding device as claimed in claim 7, wherein the holding member (2) comprises a first and a second lever element (21, 22), wherein the first lever element (21) can be rotated relative to the second lever element (22) and the holding device (1) is to be arranged on a receiving portion (3) of the syringe pump (10) in such a way that when the first lever element (21) is rotated, a force is exerted on the holding member (2) that generates a linear movement of the holding member (2).

9. Holding device as claimed in one of the claims 3 to 8, wherein the first and the second part (61, 62) of the clutching mechanism (6) realizes a form-fitted clutch.

10. Holding device as claimed in one of the claims 3 to 9, wherein the first and the second part (61, 62) of the clutching mechanism (6) form a one-way clutch.

11. Holding device as claimed in one of the preceding claims, further comprising a spring (7) that has the tendency to rotate the holding member (2) in the closed position.

12. Holding device as claimed in claims 1 and 11 wherein the spring (7) is a coil spring surrounding the shaft (4) connected to the holding member (2).

13. Syringe pump comprising a holding device (1) as claimed in one of the preceding claims.

## Patentansprüche

1. Haltevorrichtung für eine Spritzenpumpe, wobei die Haltevorrichtung (1) zum Halten des Zylinders (101) einer Spritze (102) konfiguriert ist und Folgendes umfasst:
- mindestens ein Halteglied (2), das aus einer offenen in eine geschlossene Position drehbar ist, und
- einen Blockiermechanismus, der so mit dem Halteglied (2) zusammenwirkt, dass er eine Drehung des Halteglieds (2) aus der offenen in die geschlossene Position gestattet und eine Drehung aus der geschlossenen in die offene Position blockiert,
**gekennzeichnet durch**
einen Kupplungsmechanismus (6), der ein Ausrücken des Halteglieds (2) aus dem Blockiermechanismus gestattet, so dass das Halteglied (2) aus der geschlossenen in die offene Position gedreht werden kann, und dass der Blockiermechanismus ein Einweglager (5) umfasst, über das das Halteglied (2) drehbar an einem Aufnahmeabschnitt (3) der Spritzenpumpe (10) zur Aufnahme des Spritzenzylinders (101) montiert ist,
wobei das Halteglied (2) mit einem Schaft (4) verbunden ist, der drehbar an einem Aufnahmeabschnitt (3) der Spritzenpumpe (10) zur Aufnahme des Spritzenzylinders (101) montiert ist, wobei eine Achse des Schafts (4) und eine Achse des Aufnahmeabschnitts (3) parallel zueinander verlaufen.

2. Haltevorrichtung nach Anspruch 1, wobei der Schaft (4) über das Einweglager (5) drehbar am Aufnahmeabschnitt (3) der Spritzenpumpe (10) montiert ist.

3. Haltevorrichtung nach einem der vorhergehenden Ansprüche, wobei der Kupplungsmechanismus (6) einen ersten Teil (61), der fest mit einem Aufnahmeabschnitt (3) der Spritzenpumpe (10) zur Aufnahme des Spritzenzylinders (101) verbunden ist, und einen mit dem ersten Teil (61) zusammenwirkenden zweiten Teil (62) umfasst, wobei der zweite Teil (62) so an das Halteglied (2) gekoppelt ist, dass er aus dem ersten Teil (61) ausgerückt werden kann, indem das Halteglied (2) vom ersten Teil (61) weg bewegt wird.

4. Haltevorrichtung nach Anspruch 3, wobei das Halteglied (2) durch Ausrücken des zweiten Teils (62) aus dem ersten Teil (61) aus dem Blockiermechanismus ausgerückt werden kann.

5. Haltevorrichtung nach Ansprüchen 1 und 4, wobei der zweite Teil (62) aus dem ersten Teil (61) ausgerückt werden kann, indem das Halteglied (2) entlang der Achse des Schafts (4) vom ersten Teil (61) weg bewegt wird.

6. Haltevorrichtung nach einem der Ansprüche 3 bis 5, soweit sie von Anspruch 1 abhängen, wobei das Einweglager (5) fest mit dem zweiten Teil (62) des Kupplungsmechanismus (6) verbunden ist.

7. Haltevorrichtung nach einem der Ansprüche 3 bis 6, wobei der zweite Teil (62) des Kupplungsmechanismus (6) so an das Halteglied (2) gekoppelt ist, dass er einer linearen Bewegung des Halteglieds (2) folgt.

8. Haltevorrichtung nach Anspruch 7, wobei das Halteglied (2) ein erstes und ein zweites Hebelelement (21, 22) umfasst, wobei das erste Hebelelement (21) bezüglich des zweiten Hebelelements (22) gedreht werden kann und die Haltevorrichtung (1) so an einem Aufnahmeabschnitt (3) der Spritzenpumpe (10) anzuordnen ist, dass beim Drehen des ersten Hebelelements (21) eine Kraft auf das Halteglied (2) ausgeübt wird, die eine lineare Bewegung des Halteglieds (2) erzeugt.

9. Haltevorrichtung nach einem der Ansprüche 3 bis 8, wobei der erste und der zweite Teil (61, 62) des Kupplungsmechanismus (6) eine Formschluss-Kupplung bilden.

10. Haltevorrichtung nach einem der Ansprüche 3 bis 9, wobei der erste und der zweite Teil (61, 62) des Kupplungsmechanismus (6) eine Einwegkupplung bilden.

11. Haltevorrichtung nach einem der vorhergehenden Ansprüche, ferner umfassend eine Feder (7), die dazu neigt, das Halteglied (2) in die geschlossene Position zu drehen.

12. Haltevorrichtung nach Ansprüchen 1 und 11, wobei die Feder (7) eine Spiralfeder ist, die den mit dem Halteglied (2) verbundenen Schaft (4) umgibt.

13. Spritzenpumpe umfassend eine Haltevorrichtung (1) nach einem der vorhergehenden Ansprüche.

## Revendications

1. Dispositif de support pour une pompe à seringue, le dispositif de support (1) étant configuré pour supporter le cylindre (101) d'une seringue (102) et comprenant :
- au moins un élément de support (2) qui est rotatif d'une position ouverte à une position fermée ; et
- un mécanisme de blocage interagissant avec l'élément de support (2) de telle sorte qu'il permet une rotation de l'élément de support (2) de la position ouverte à la position fermée et bloque une rotation de la position fermée à la position ouverte,
**caractérisé par** un mécanisme d'embrayage (6) permettant un désaccouplement de l'élément de support (2) vis-à-vis du mécanisme de blocage de telle sorte que l'élément de support (2) puisse tourner de la position fermée à la position ouverte, et en ce que le mécanisme de blocage comprend un palier unidirectionnel (5) par le biais duquel l'élément de support (2) est installé à rotation sur une partie de réception (3) de la pompe à seringue (10) destinée à recevoir le cylindre de seringue (101),
l'élément de support (2) étant raccordé à un arbre (4) qui est installé à rotation sur une partie de réception (3) de la pompe à seringue (10) destinée à recevoir le cylindre de seringue (101), un axe de l'arbre (4) et un axe de la partie de réception (3) étant parallèles l'un à l'autre.

2. Dispositif de support selon la revendication 1, dans lequel l'arbre (4) est installé à rotation sur la partie de réception (3) de la pompe à seringue (10) par le biais du palier unidirectionnel (5).

3. Dispositif de support selon l'une des revendications précédentes, dans lequel le mécanisme d'embrayage (6) comprend une première partie (61) raccordée de manière fixe à une partie de réception (3) de la pompe à seringue (10) destinée à recevoir le cylindre de seringue (101), et une seconde partie (62) interagissant avec la première partie (61), la seconde partie (62) étant reliée à l'élément de support (2) de telle sorte qu'elle puisse être désaccouplée de la première partie (61) en éloignant l'élément de support (2) de la première partie (61).

4. Dispositif de support selon la revendication 3, dans lequel l'élément de support (2) peut être désaccouplé du mécanisme de blocage en désaccouplant la seconde patrie (62) de la première partie (61).

5. Dispositif de support selon les revendications 1 et 4, dans lequel la seconde partie (62) peut être désaccouplée de la première partie (61) en éloignant l'élément de support (2) de la première partie (61) le long de l'axe de l'arbre (4).

6. Dispositif de support selon l'une des revendications 3 à 5 dans la mesure où elles dépendent de la revendication 1, dans lequel le palier unidirectionnel (5) est raccordé de manière fixe à la seconde partie (62) du mécanisme d'embrayage (6).

7. Dispositif de support selon l'une des revendications 3 à 6, dans lequel la seconde partie (62) du mécanisme d'embrayage (6) est reliée à l'élément de support (2) de telle sorte qu'elle suive un déplacement linéaire de l'élément de support (2).

8. Dispositif de support selon la revendication 7, dans lequel l'élément de support (2) comprend des premier et second éléments formant leviers (21, 22), le premier élément formant levier (21) pouvant être tourné par rapport au second élément formant levier (22) et le dispositif de support (1) étant destiné à être disposé sur une partie de réception (3) de la pompe à seringue (10) de telle sorte que, lorsque le premier élément formant levier (21) est tourné, une force soit exercée sur l'élément de support (2), ladite force générant un déplacement linéaire de l'élément de support (2).

9. Dispositif de support selon l'une des revendications 3 à 8, dans lequel les première et seconde parties (61, 62) du mécanisme d'embrayage (6) constituent un embrayage à complémentarité de forme.

10. Dispositif de support selon l'une des revendications 3 à 9, dans lequel les première et seconde parties (61, 62) du mécanisme d'embrayage (6) forment un embrayage unidirectionnel.

11. Dispositif selon l'une des revendications précédentes, comprenant en outre un ressort (7) qui a tendance à faire tourner l'élément de support (2) vers la position fermée.

12. Dispositif de support selon les revendications 1 et 11, dans lequel le ressort (7) est un ressort hélicoïdal entourant l'arbre (4) raccordé à l'élément de support (2).

13. Pompe à seringue comprenant un dispositif de support (1) selon l'une des revendications précédentes.
